# EUROPEAN PATENT SPECIFICATION

(11) **EP 2 539 032 B1**
(45) Date of publication and mention of the grant of the patent: **20.04.2016**
(21) Application number: 10732259.6
(22) Date of filing: 28.02.2010
(51) Int. Cl.: B01D 11/02, A61K 8/97, A61Q 19/00

(54) **A METHOD OF EXTRACTING PLANT SUBSTANCES, THE EXTRACT AND ITS USE AND CORRESPONDING DEVICE**
VERFAHREN ZUR EXTRAKTION VON PFLANZENSTOFFEN, EXKTRAKT UND SEINE VERWENDUNG SOWIE ENTSPRECHENDE VORRICHTUNG
METHODE D'EXTRACTION DE SUBSTANCES VEGETALES, EXTRAIT ET SON UTILISATION AINSI QU'UN DISPOSITIF CORRESPONDANT

(43) Date of publication of application: 02.01.2013
(73) Proprietor: Primrose A.S., 110 00 Prague 1 (CZ)
(72) Inventor: VANA, Radek, 286 01 Caslav (CZ)
(74) Representative: Fischer, Michael
(86) International application number: PCT/CZ2010/000023
(87) International publication number: WO 2011/103843

(56) References cited:
- EP-A1- 1 469 927
- JP-A- 2 062 827
- JP-A- 62 146 581
- JP-A- 2005 289 950
- US-A- 5 026 549
- US-A1- 2006 198 811

## Description

### Field of the invention

The invention relates to a method of extracting plant substance into a final extract according to EP-1469927 of the same applicant.

According to the European patent EP-1469927 B1 of the same applicant is known a process of extracting plant substances into a final extract, which is pasteurised and conserved, whereas the plant substance blend is periodically extracted in extraction periods composed of a first phase, in which the plant substance blend in the first filtration bag is partially extracted with water into a partial extract, and of a second phase, in which the plant substance blend in the second filtration bag is extracted with the partial extract of the first phase into a final phase, whilst in the second filtration bag a new plant. substance blend is extracted in all extraction periods and in the first filtration bag a new plant substance blend is extracted in the initial extraction period and a partially extracted plant substance blend from the second phase of the foregoing extraction period is extracted in the subsequent operational extraction periods.

In the initial extraction period a new plant substance blend in the first filtration bag is extracted, using water, in the first extraction vessel into a partial extract. The partial extract is transferred to the second extraction vessel with a second filtration bag filled with a new plant extracts blend, which is extracted in the second extraction vessel by the partial extract into a final extract. In accordance with EP 1469927 the extraction is carried out with forced circulation of water and of partial extract, the temperature of the extraction media at the entry to the extraction vessel is kept at 45 - 55° C, the extraction medium is kept in the extraction vessels for 15 - 25 minutes and the mixture of plant substance is extracted with water having a modified pH acidity factor, whilst the water's acidity is kept between pH 6 - 8. According to the European Patent EP 1469927 B1, of the same applicant, an equipment for production of extracts of plant substances is known, which includes at least two circuits, in which an extraction vessel containing a filtration bag, a circulation pump and a heat exchanger are connected one after the other. The circuits are interconnected to one another, and linked both to a water container and to a container for the final product.

The method of extraction plant substance in accordance with EP 1469927 provides a pure and quality final product, which does not require any further modifications, in particular no filtration, no acidity adjustment and no repeated heating, however it does provide a final product at a large energy demand and it is desirable to reduce this. The intention's objective is to increase the efficiency and yield of the plant substance extraction method according to EP 1469927 and shorten the period of extracting plant substance compared to EP 1469927.

A method of extracting plant substance is known from the document US5026549 according to which the plant material is treated by adjustably and continuously feeding a vapour to achieve a hydrolysis or hydro-distillation of the plant material. A partial extraction of the plant material is provided by counter flowing steam in an open tank whereas a substance (essential oil from the volatile fractions of the fresh plant material) is separated from the plant material and conducted from the open tank out to a condenser. The water vapour is a carrier of the resultant liquid containing volatile fractions with essential oil. The separation of the substance by means of vapour is, however, a very aggressive and invading action which damages the quality of the plant substances of the plant material.

### Summary of the invention

The present invention provides a method of extraction plant substances into a final extract, which is pasteurised and conserved, according to which the plant substance blend is periodically extracted in extraction periods composed of a first phase, in which the plant substance blend in the first filtration bag is partially extracted with water into a partial extract, and of a second phase, in which the plant substance blend in the second filtration bag is extracted with the partial extract of the first phase into a final extract, whilst in the second filtration bag a new plant substance blend is extracted in all extraction periods and in the first filtration bag a new plant substance blend is extracted in the initial extraction period and a partially extracted plant substance blend from the second phase of the foregoing extraction period is extracted in the subsequent operational extraction periods, according to the invention, the nature of which consisting in that the new plant substance blend undergoes a treatment of Water vapour before extraction.

According to another embodiment the new plant substance blend undergoes a treatment of water vapour, which is a component of a vapour mix.

According to another embodiment the new plant substance blend undergoes a treatment of water vapour at a pressure of 1 to 10 bar, preferably 3 to 6 bar, for a period of 0.5 to 10 minutes, preferably 3 to 5 minutes.

According to another embodiment the new mixture of plant substance undergoes the treatment of water vapour in the second filtration bag in all of the extraction periods and in the first filtration bag in the initial extraction period.

The present invention further provides a device for extracting plant substances comprising circuits in which extraction vessels containing filtration bags are placed in series with circulation pumps and heat exchangers and further comprising a water container with water supply and an inlet for pH value adjustment and a final product container, whereas the circuits are connectable mutually and with the water reservoir and with the final products container, and according to the invention, the extraction vessel is connected with a vapour supply pipe and an vapour discharge pipe, provided by a stop valve.

The extract of plant substances prepared in a method according to the present invention may be used as a component of a pharmaceutical preparation, a food product, a drug-store product, a feedstuff or fertilisers.

Disrupting the plant substance with vapour increases the effect of the subsequent extraction by extracting treatment agent - water, because it is easier to extract the disrupted plant parts. It significantly shortens the period of the subsequent extraction and increases the yield of the active substances. A consequence of applying vapour to the new mixture of plant substance before extraction is to shorten the subsequent extraction process by almost a half. Apart from shortening the subsequent extraction period the application of vapour increases the yield of the active substances by roughly 30 %, in particular those substances falling under the titles of alkaloids, flavonoids, essential oils, terpenes, microelements and glycosides.

### Brief description of the drawings

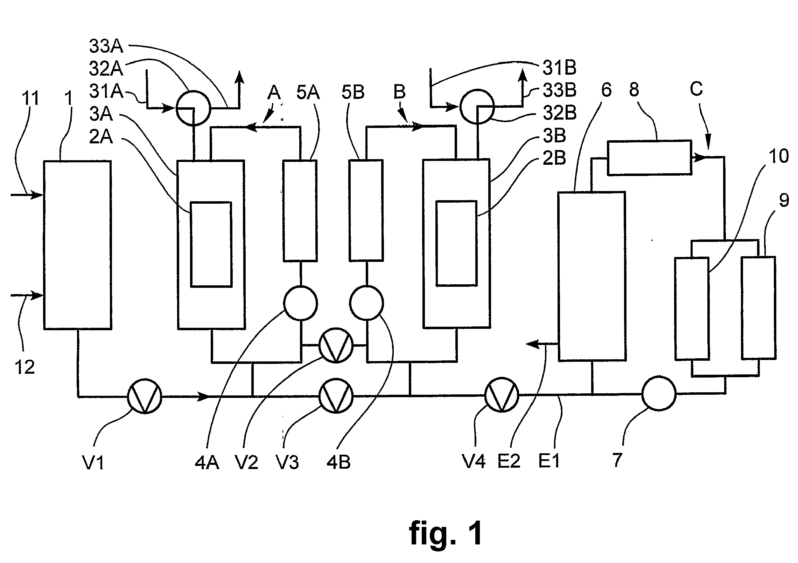

The invention will be clarified in detail using the drawings, wherein Fig. 1 presents a block diagram of a device for extracting plant substances according to the invention.

### Detailed description of the preferred embodiments

When extracting plant substances, in accordance with the invention, a new mixture of plant substance is placed in the filtration bag and the filtration bag is placed into the extraction vessel, which is closed with a lid. Water vapour is then released into the extraction vessel either on its own or in a mix of other vapours. The most suitable value for the water vapour pressure is between 3 to 6 bar. The time for the water vapour to act upon the new plant substance mixture ranges from 0.5 to 10 minutes, the most suitable time period is 3 to 5 minutes. The time chosen depends on whether the extraction is performed on the leaves, stems, flowers or roots of the plant substance. Tough and hard plant parts undergo the actions of water vapour for a longer period than soft plant parts. Tough parts, such as roots, require longer for the vapour to take effect. The steam disrupts the stem's epidermis (protoderm) and the ground tissue (collenchyma, sclerenchyma, parenchyma) and opens up the veins. For roots, vapour disrupts the epidermis, the endodermis, the pericycle and the veins. In leaves it disrupts the cuticle, expands the stomata and disrupts the mesenchyme and tissue. After the action of water vapour has ceased the extraction agent is introduced to the extraction vessel - water in the initial extraction period or partial extract in the operational extraction period. Afterwards the water or partial extract circulate in the extraction vessel for roughly 5 - 10 minutes whereas the extraction of the plant substances is carried out, which are placed in the filtration bag. With regards to the fact that the extraction of plant substance takes place much quicker than according to the method for extracting plant substance in EP 1469927, the extraction agent, water, and the partial extract can flow faster around the extraction vessel and multi-speed pumps can be used, which allow to accelerate the circulation of the water and of the partial extract in the circuits.

According to the invention, plant substance can be extracted in equipment that, according to fig. 1, incorporates circuits A and B containing extraction vessels (3A, 3B) that have filtration bags (2A, 2B) connected one after another with circulation pumps (4A, 4B) and heat exchangers (5A, 5B). The equipment further includes a water container (1) with a water inlet (11) and an opening (12) to regulate the pH value and a container (6) for the final product. Circuits A and B can be mutually connected and also connected with the water container (1) and the container for the final product (6). The water container (1) is connected through the stop-valve (V1) in parallel with two circuits (A, B) for phase extraction of the plant substance mixture. Both circuits (A, B) contain the first and second extraction tank (3A, 3B) in which the filtration bags are located (2A, 2B). Inlet tubes with stop-valves (31A, 31B) for steam to enter into the extraction tanks (3A, 3B). The stop-values (32A, 32B) are three-way enabling in the first position the inflow of pressurised steam through the inlet tubes (31 A, 31B), in the second position closing of the valve and in the third position the pressurised steam to be released through discharge pipes (33a, 33B).

In circuits A and B the extraction tanks are arranged one after the other circulation pumps (4A, 4B) and heat exchangers (5A, 5B). Circuits A and B are on the same level as the bottom of the extraction vessels (3A, 3B) mutually connected through the stop-valve (V2). Circuits A and B are further connected by a stop-valve (V3), whilst both circuits can be connected by a stop-valve (V3) with connection piping E1 for draining the final extract, in which there is a stop-valve (V4) . The connection piping E1 is connected through a stop-valve (V4) to a container for the final product, which has an outlet (E2) to let the final product, out.

According to the invention the method for extracting plant substance takes place both in the initial run-up period and in the operational periods.

In the initial period the new mixture of plant substance is put into the filtration bags (2A, 2B), which are placed into the first and second extraction vessels (3A and 3B). The lids to extraction vessels 3A and 3B are shut and water vapour is released into the extraction vessels (3A, 3B) through the inlet tubes (31A, 31B). The water vapour is kept between 1 to 10 bar with the ideal range being 3 to 6 bar. The water vapour is left to take effect on the mixture of plants substance placed in the filtration bags (2A, 2B) for 0.5 to 10 minutes, with the ideal time period being 3 to 5 minutes. After the chosen time period has passed the vapour is released from vessel 3A and 3b and the extraction agent, water, is released into vessel 3A and the extraction process begins.

In the initial run-up period the first phase takes place in the first extraction vessel 3A, by water extraction of the new mix of plant substance, which have undergone the effects of water vapour. The first phase of extraction takes 5 - 10 minutes. After the first phase is complete the partial extract formed is released into the second extraction vessel 3B, where the second extraction phase takes place during which the partial extract circulates as an extractant through filtration bag 2B, in which a new mixture of plant substance has been placed after undergoing the action of water vapour. The second phase of extraction also takes about 5 - 10 minutes. After the second phase is complete the final extract is released from the extraction vessel 3B into the container for the final product (6). This ends the initial extraction period.

Afterwards the operational periods of phase extraction follow.

In the operational period, in parallel with the second extraction phase in the second extraction vessel 3B, the first extraction phase of the mixture of plant substance extracted using water is carried out in the first extraction vessel 3A. After the second extraction phase ends in the second extraction vessel 3B, the filling of the filtration bag 2B, is changed for a new mixture of plant substance.

The second extraction vessel 3B is closed, water vapour is released into it and left to act on the new mixture of plant substance under a pressure of 1 to 10 bar with the best pressure being 3 to 6 bar, for 0.5 to 10 minutes, the best time being 3 to 5 minutes. After the time period chosen for the water vapour treatment, the water vapour is released from extraction vessel 3B and the extraction agent - the partial extract from vessel 3A - is pumped into vessel 3B and the second stage of the operational period of extraction starts in vessel 3B.

After the second extraction phase in the second extraction vessel 3B ends, the final extract is pumped into the final product container 6. Meanwhile the first extraction phase takes place in the first extraction vessel 3A, the partial extract is pumped into the second extraction vessel 3B, the mixture of plant substance in filtration bag 2A in extraction vessel 3A is exchanged for the partially leached mixture of plant substance in the second extraction vessel 3B and then the first phase of extraction with water takes place in the first extraction vessel 3A.

The method of extracting plant substance in accordance with the invention can be altered so that the flow direction of the water extractant and the partial extract extractant can be reversed when the first and second phase of extraction in the extraction vessels are both carried out. The said process of production of extracts of the plant substances can be modified so that it takes place only the one vessel. Before the extraction of the new mixture of plant substance the new mixture of plant substance always undergoes the effect of water vapour for the same time period and under the same pressure as mentioned above. Various other modifications of the method for extracting plant substance in accordance with the invention and of the equipment for carrying out this method, as presented in file EP 1469928, are possible. For further processing of the final product and for use of the extract the principles presented in file EP 1469928 are valid.

### Example 1.

According to the known method of extraction of plant substance, a mixture of plant substance (relaxation mix) containing hops, stinging nettle, sweet woodruff, lavender was extracted without the effect of steam and a dry weight of 2.2 %, a density of 0.970 g/cm3 and an acidity of 5.0 - 5.5 pH were achieved.

Extraction was performed on the same mixture of plant substance using the method in accordance with the invention and increase of the dry weight content to 3 % (increased content of active substances) was detected, the density rose to 1.200 g/cm3 and the acidity rose by one degree.

### Example 2.

According to the known method of extraction of plant substance a mixture of plant substance (detoxification mix) containing eyebright, rooibos, common agrimony, common mallow was extracted without the effect of steam, and a dry weight of 2.3 %, a density of 0.980 g/cm3 and an acidity of 5.0 - 6.0 pH were achieved.

Extraction was performed on the same mixture of plant substance using the.method in accordance with the invention and increase of the dry weight content to 3 % (increased content of active substances) was detected, density rose to 1.200 g/cm3 and acidity rose by one degree.

### Example 3.

According to the known method of extraction of plant substance a mixture of plant substance (stimulation mix) containing silver birch, pot marigold, common horsetail, peppermint was extracted without the effect of steam, and a dry weight of 2.1 %, a density of 0.980 g/cm3 and an acidity of 4.4 - 5.4 pH were achieved.

Extraction was performer on the same mixture of plant substance using the method in accordance with the invention and increase of the dry weight content to 3 % (increased content of active substances) was detected, density rose to 1.200 g/cm3 and acidity rose by one degree.

### Example 4.

According to the known method of extraction of plant substance a mixture of plant substance (dermatological mix) containing common oat, great burdock, wild thyme, buckwheat was extracted without the effect of steam, and a dry weight of 2.1 %, a density of 0.960 g/cm3 and an acidity of 5.0 - 6.0 pH were achieved.

Extraction was performed on the same mixture of plant substance using the method in accordance with the invention and an increase of the dry weight content to 3 % (increased content of active substances) was detected and density rose to 1.200 g/cm3 and acidity rose by one degree.

The extract of plant substances prepared in the method according to the invention brings advantages in the manufacture of pharmaceutical preparations, food and drug-store products (e.g. cosmetics, cleaning agents and detergents) for both people and animals, feeds and fertilizers.

The feeds made using the extract obtained by extraction of the plant substance in.the manner according to the invention cause animals to have a higher food intake and subsequent weight gain. For instance a 2.5% by weight nettle extract was prepared and used as a component of a feed mix at 3 % by weight. The usual period for slaughterhouse feed of 111 days was shortened by 11 days to 100 days. Thus the animals' weight increase was 10 % quicker.

The fertilizers made with an extract obtained by extraction from plant substance in the manner according to the invention caused unprecedented plant growth. An aqueous extract of nettle, tobacco and birch can be used as a universal liquid fertilizer. It gives accelerated growth, greater resistance and faster plant germination. For instance an extract from nettle and birch at a ratio of 50:50 % with a dry weight of 1.6 % was prepared and used for radish (*Raphanus sativus*) at a dosage of 10 ml of extract per 1 litre of water as a liquid fertiliser applied to both roots and leaves. The plants germinated 7 days earlier thus shortening the growing season of roughly 70 days by 1.0%.

Increasing the effects compared to the current state was demonstrated when using the extract of plant substance obtained according to the invention in pharmaceutical preparations of phyto-pharmacy as a raw ingredient for medicines and syrups. For manufacturing pharmaceutical products a gel or syrup made from an aqueous extract of comfrey, camomile, and eyebright can be used. Clear positive effects were discovered for the joints, against inflammations, against arthritis, for increasing immunity, against colds, etc. The advantage is that the effective substances from the herbs, obtained in the extract, cross over directly into the product. For instance an extract was made from comfrey and added to a gel against joint inflammation at 5 - 10 % by weight. The start of the effect of the gel was accelerated from usual 2-3 days to 1 day only.

Likewise using the extract from plant substance in foodstuffs and food supplements, such as beverages, spreads and mayonnaises, was proven to increase the energy effects of such foods and food supplements. An aqueous extract of onion, garlic, oregano, rosemary and basil can be used as an ingredient in the food industry and mixed into mayonnaise or a butter spread. A more distinctive, absolutely natural taste is achieved without using perfumes or chemical preparations. Energy drinks, such as lemonade or ice tea, can be produced from an aqueous extract of herb mixes. For instance extracts were made from onion, garlic and leek with a dry weight of 1.7 % and each one was used in butter spreads at 5 % by weight. A far better taste was achieved without using any of the otherwise usual chemical additives.

Cleaning agents and detergents made using an extract obtained by extracting plant compounds in accordance with the invention have better cleaning abilities, their antiseptic and antibacterial effects were increased and the effect on the skin was better. A suitable combination for extraction is thyme, camomile, marigold and common soapwort.

Cosmetic products made using an extract obtained by extracting plant compounds in accordance with the invention can rejuvenate the hair matrix, reduce dandruff formation and soften the skin. It is suitable to use an extract of birch, camomile or nettle and use the aqueous extract to produce shampoo, shower gel or a hair tonic using 100 % of the extract in the product.

Baths made using an extract obtained by extracting plant compounds in accordance with the invention can have a pleasant action increasing the effect without perfumes and dyes, the bath solution does not contain alcohol, oil or any other organic solvent and does not soil the bath. For baths it is suitable to use an extract of lavender, mint and comfrey at 100 % concentration of the extract in the bath.

## Claims

1. Method of extraction plant substances into a final extract, which is pasteurised and conserved, according to which a plant substance blend is periodically extracted in extraction periods composed of a first phase, in which the plant substance blend in a first filtration bag is partially extracted with water into a partial extract, and of
a second phase, in which the plant substance blend in a second filtration bag is extracted with the partial extract of the first phase into a final extract, whilst in the second filtration bag a new plant substance blend is extracted in all extraction periods and in the first filtration bag a new plant substance blend is extracted in the initial extraction period and a partially extracted plant substance blend from the second phase of the foregoing extraction period is extracted in the subsequent operational extraction periods,
**characterized in that** before the extraction , the new plant substance blend undergoes a disruption of the inner parts of the stems or roots or leaves of the plant substance blend by treatment by water vapour in a closed vessel at a pressure of 1 to 10 bar, preferably 3 to 6 bar for a determined time period of 0,5 to 10 minutes, preferably 3 to 5 minutes, after the determined time period has passed the vapour is released from the vessel.

2. Method of extraction plant substances according to the claim 1,
**characterised by** that the new plant substance blend undergoes a treatment of water vapour, which is a component of a vapour mix.

## Patentansprüche

1. Verfahren der Extraktion pflanzlicher Stoffe zum Finalextrakt, der pasteurisiert und konserviert wird, wonach das Gemisch pflanzlicher Stoffe periodisch in Extraktionsperioden extrahiert wird, die aus einer ersten Phase bestehen, in der das Gemisch pflanzlicher Stoffe in dem ersten Filtrationsbeutel teilweise mit Wasser zu einem Teilextrakt extrahiert wird, und aus einer zweiten Phase, in der das Gemisch pflanzlicher Stoffe im zweiten Filtrationsbeutel mit dem Teilextrakt der ersten Phase zum Finalextrakt extrahiert wird,
wobei in dem zweiten Filtrationsbeutel das neue Gemisch pflanzlicher Stoffe in allen Extraktionsperioden extrahiert wird und im ersten Filtrationsbeutel in der anfänglichen Extraktionsperiode das neue Gemisch pflanzlicher Stoffe und in den nachfolgenden betrieblichen Extraktionsperioden das teilweise extrahierte Gemisch pflanzlicher Stoffe der zweiten Phase der vorherigen Extraktionsperiode extrahiert werden,
**gekennzeichnet dadurch, dass**
das neue Gemisch pflanzlicher Stoffe sich vor der Extraktion dem Zerbrechen der Innenteile der Stiele oder Wurzeln oder Blätter des Gemisches pflanzlicher Stoffe durch Einwirken von Wasserdampf in einem abgeschlossenen Behälter bei einem Druck von 1 bis 10 bar, mit Vorteil 3 bis 6 bar, für die Dauer von 0,5 bis 10 Minuten, mit Vorteil 3 bis 5 Minuten unterzieht, nach Ablauf der gewählten Zeitperiode der Dampf aus dem Behälter abgelassen wird.

2. Verfahren der Extraktion pflanzlicher Stoffe nach Anspruch 1,
**gekennzeichnet dadurch, dass**
das neue Gemisch der pflanzlichen Stoffe sich der Einwirkung von Wasserdampf unterzieht, welcher eine Komponente des Dampfgemisches ist.

## Revendications

1. Procédé d'extraction des substances végétales à un extrait final qui est pasteurisé et conservé et selon lequel un mélange des substances végétales est périodiquement lixivié au cours des périodes d'extraction composées d'une première phase au cours de laquelle le mélange des substances végétales disposé dans le premier sac de filtration est partiellement lixivié avec de l'eau à un extrait partiel et d'une deuxième phase au cours de laquelle le mélange de substances végétales disposé dans un deuxième sac de filtration est lixivié moyennant de l'extrait partiel de la première phase jusqu'au produit final d'extraction,
cependant que dans le second sac de filtration est lixivié au cours de toutes les périodes de lixiviation un nouveau mélange des substances végétales et dans le premier sac de filtration est lixivié au cours de la période initiale de lixiviation un nouveau mélange des substances végétales et au cours des périodes suivantes d'exploitation de lixiviation, le mélange partiellement lixivié des substances végétales de la seconde phase de la période précédente de lixiviation,
**caractérisée par le fait que**
avant la lixiviation le nouveau mélange des substances végétales est soumis à une déformation des parties intérieures des tiges ou des racines ou des feuilles du mélange des substances végétales moyennant d'un traitement de la vapeur d'eau dans un récipient fermé sous une pression de 1 à 10 bars, de préférence de 3 à 6 bars, pendant 0,5 à 10 minutes, de préférence de 3 à 5 minutes et après la fin de la période du temps sélectionnée la vapeur soit évacuée du récipient.

2. Procédé de lixiviation des substances végétales selon la revendication 1,
**caractérisée par le fait que**
le nouveau mélange des substances végétales est soumis à l'exposition de la vapeur d'eau, ce qui est une composante des mélanges de vapeur.
